Europäisches Patentamt

European Patent Office

Office européen des brevets

(19)

(11) **EP 0 888 189 B1**

(12) **EUROPÄISCHE PATENTSCHRIFT**

(45) Veröffentlichungstag und Bekanntmachung des Hinweises auf die Patenterteilung:
**15.09.1999 Patentblatt 1999/37**

(21) Anmeldenummer: **97915363.2**

(22) Anmeldetag: **13.03.1997**

(51) Int. Cl.$^6$: **B01J 37/34**, B01J 37/02, B01J 37/00

(86) Internationale Anmeldenummer:
**PCT/EP97/01279**

(87) Internationale Veröffentlichungsnummer:
**WO 97/34695 (25.09.1997 Gazette 1997/41)**

(54) **VERFAHREN ZUM HERSTELLEN EINES HETEROGENEN KATALYSATORS**

PROCESS FOR MANUFACTURING A HETEROGENOUS CATALYST

PROCEDE DE FABRICATION D'UN CATALYSEUR HETEROGENE

(84) Benannte Vertragsstaaten:
**BE DE FR GB NL**

(30) Priorität: **21.03.1996 DE 19611132**

(43) Veröffentlichungstag der Anmeldung:
**07.01.1999 Patentblatt 1999/01**

(73) Patentinhaber: **Pohl, Joachim**
**14513 Teltow (DE)**

(72) Erfinder: **Pohl, Joachim**
**14513 Teltow (DE)**

(74) Vertreter: **Schoenen, Norbert**
**postfach 10 22 09**
**47412 Moers (DE)**

(56) Entgegenhaltungen:
**WO-A-94/15708**

- **PATENT ABSTRACTS OF JAPAN vol. 011, no. 089 (C-411), 19.März 1987 & JP 61 242644 A (TOYOTA MOTOR CORP), 28.Oktober 1986,**
- **PATENT ABSTRACTS OF JAPAN vol. 001, no. 026 (C-007), 26.März 1977 & JP 51 137686 A (NIPPON MINING CO LTD), 27.November 1976,**

**Beschreibung**

Die Erfindung betrifft ein Verfahren zum Herstellen eines heterogenen, massiven Katalysators, der aus mindestens einer katalytisch aktiven und mindestens einer katalytisch inerten Komponente besteht, wobei die Komponenten ineinander dispergiert sind.

Der in dieser Anmeldung genannte "mittlere Korndurchmesser" bzw. die "Korngröße" versteht sich als $d_{50}$-Wert. Dieser Wert ist so definiert, daß 50 % der betrachteten Partikel einen kleineren Korndurchmesser als den angegebenen $d_{50}$-Wert haben.

Bei der Herstellung industriell eingesetzter, heterogener Katalysatoren werden zwei Arten unterschieden, nämlich Trägerkatalysatoren und massive Katalysatoren. Bei Trägerkatalysatoren ist das katalytisch aktive Material, zum Beispiel Metallsalze oder Metalloxide, auf einem katalytisch inaktiven Träger, zum Beispiel Aluminiumoxid, durch Tränken oder Imprägnieren aufgebracht. Die Form und Größe des Trägers, z. B. Kugeln oder Tabletten mit Abmessungen von 10 bis 50 mm, bestimmen in diesem Fall die Form und Größe des fertigen Katalysators. Im Gegensatz dazu besteht der massive Katalysator aus einem Pulvergemisch einer katalytisch aktiven und einer inerten Masse. Er erhält seine Form und Größe durch eine abschließende Formgebung dieses Gemisches, z. B. durch Extrudieren oder Pelletieren.

Durch das erfindungsgemäße Verfahren werden massive Katalysatoren hergestellt. Sie werden im Stand der Technik durch Ausfällen einer Metallsalzlösung, Filtration, Trocknung, Kalzinierung und anschließende Formgebung und gegebenenfalls Reduktion, z. B. mit Wasserstoff, hergestellt.

Die Aktivität, Selektivität und Lebensdauer eines Katalysators hängen bei festgelegten chemischen Grundzusammensetzungen zum großen Teil von der physikalischen Struktur ab.

Hierunter ist die Dispersität, die Struktur der Oberfläche und die Porenstruktur zu verstehen. Dementsprechend wird für viele Anwendungsbereiche ein feinkörniger, innerhalb des fertig geformten Katalysators fein verteilter Katalysator mit einer hohen spezifischen Oberfläche bevorzugt.

Um eine besonders hohe Dispersität sowie Oberfläche des massiven Katalysators zu erhalten, ist es im Stand der Technik bekannt, die katalytisch aktiven Metallsalze zusammen mit katalytisch inerten Komponenten gemeinsam auszufällen. Es sei ausdrücklich darauf hingewiesen, daß es sich bei diesen Katalysatoren nicht um Trägerkatalysatoren, sondern um massive Katalysatoren handelt, die durch eine sogenannte "Mischfällung" hergestellt werden.

Ein Beispiel für einer derartige Mischfällung findet sich in der DE 39 30 288 A1. Hier wird ein massiver Kupfer-Zink-Silikat-Katalysator durch Zugabe einer Lösung von Kupfernitrat und Zinknitrat zu einer Lösung von Natriumsilicat unter starkem Rühren hergestellt.

Wichtig bei einer solchen Mischfällung ist ein größeres Löslichkeitsprodukt der aktiven Komponente als das der inerten Komponente beim vorliegendem pH-Wert. In diesem Fall fällt zunächst die inerte Komponente in feinen, typischerweise etwa 500 nm großen Partikeln aus, auf die dann die kleineren Partikel der aktiven Komponente aufwachsen. Die aus größeren, inerten Teilchen mit angelagerten kleinen, aktiven Teilchen bestehenden Partikel ermöglichen eine Feinverteilung der aktiven Komponente im massiven Katalysator. Diese Partikel haben außerdem eine hohe mechanische Stabilität, so daß sich die Teilchen der aktiven Komponente von den Teilchen der Inertkomponente unter üblichen Bedingungen nicht mehr trennen lassen.

Unerwünscht dagegen wären die gegenteiligen Verhältnisse der Löslichkeitsprodukte. In diesem Fall würde zunächst die aktive Komponente in relativ großen Partikeln ausfallen, auf denen sich dann die inerten feinkörnigen Partikel anlagern würden. Zum einen wird die katalytisch wirksame Oberfläche durch die Inertpartikel teilweise abgedeckt. Zum anderen liegt ein weiterer Nachteil in der experimentell festgestellten geringeren Haftung der Aktivteilchen an den Inertteilchen im Mischpartikel.

Die Mischfällung unter den oben genannten günstigen Bedingungen zeigt einige Nachteile. Zum einen liegt ein ausreichender Unterschied der Löslichkeitsprodukte von aktiver und inerter Komponente entsprechend der oben genannten Forderung nur in begrenzten Bereichen des pH-Werts vor. Der pH-Wert ändert sich jedoch während des Ausfällens, so daß in vielen Fällen nur zu Beginn der Ausfällung, aber nicht gegen Ende der Fällung ein ausreichender Unterschied der Löslichkeitsprodukte der beiden Komponenten vorliegt. Oft haben sich daher gegen Ende der Ausfällung die Löslichkeitsprodukte der beiden Komponenten soweit angenähert, daß beide Komponenten praktisch gleichzeitig ausfallen und ein Aufwachsen der Aktivkomponente auf dem vorher ausgefälltem Trägerkristalliten nicht mehr stattfindet.

Ein weiterer Nachteil liegt in der Einschränkung der eingesetzten Komponenten auf solche mit geeigneten Unterschieden der Löslichkeitsprodukte. So muß das Löslichkeitsprodukt der inerten Komponente kleiner als $10^{-9}$ sein. Eine Mischfällung mit Titandioxid als inerter Komponente, das ein Löslichkeitsprodukt von $10^{-5}$ hat, und mit Kupfer als aktiver Komponente ist daher nicht möglich.

Neben der Fällung bzw. der Mischfällung sind im Stand der Technik alternative Verfahren zur Herstellung von heterogenen, massiven Katalysatoren bekannt. Die erforderliche Feinverteilung der aktiven Komponente wird hier nicht auf physikalisch-chemischem Wege, sondern durch mechanische Zerkleinerung erreicht. So werden heterogene Katalysatoren auf der Basis nanokristalliner Legierungen durch mechanisches Legieren hergestellt (WO 90/09846 A1). Die kupfer- und nickelhaltigen Katalysatoren enthalten als Inertkomponente Silicium bzw. Siliciumdioxid. Ein besonderer Vorteil dieses Katalysators liegt in seinem abwasserfreiem Herstellungsverfahren. Zur Herstellung werden die entsprechenden metallischen Pulver in einer Kugelmühle mit hoher Energie vermahlen. Zusätzlich zur Vermahlung findet ein Verschweißen der Metallpulverpartikel untereinander statt. Verantwortlich für die dabei entstehenden Legierungen ist eine diffusionsgesteuerte Festkörperreaktion zwischen den dünnen ausgewalzten Schichten. Man erhält Kristallitgrößen unter 10 nm.

Die Herstellung von Inertkomponenten enthaltenden Katalysatoren entsprechend dem eingangs genannten Verfahren wird außerdem in der DE 43 08 120 A1 sowie der WO 94/15708 A1 beschrieben. Die DE 42 09 292 A1 beschreibt ein Verfahren zum Aufarbeiten verbrauchter Katalysatoren, wobei in ähnlicher Weise vorgegangen wird.

Nach der DE 43 08 120 A1 stellt man den Katalysator her, indem man die als Festkörper vorliegenden und den Katalysator bildenden oxidischen Ausgangsstoffe, bei denen es sich neben Kupferoxid oder einem anderen Metalloxid mindestens noch um ein weiteres Metalloxid, zum Beispiel Aluminiumoxid, handelt, miteinander vermischt und gleichzeitig oder anschließend die Oxide auf eine Korngröße kleiner als 10 $\mu$m mechanisch zerkleinert. Bevorzugt liegt der $d_{50}$-Wert der zerkleinerten Ausgangsstoffe zwischen 0,1 $\mu$m und 1 $\mu$m.

Bei den zu zerkleinernden Ausgangsstoffen kann es sich nach den Ausführungsbeispielen dieser Druckschrift um Kupferoxid und Titandioxid, bzw. um Kupferoxid und Aluminiumoxid handeln, also zum einen um eine katalytisch aktive und zum anderen um eine katalytisch inerte Komponente. Nach einer bevorzugten Ausführungsform des bekannten Verfahrens wird bei der Zerkleinerung eine Flüssigkeit, insbesondere Wasser hinzugefügt. Es werden jedoch nicht getrennte Suspensionen der katalytisch aktiven und der katalytisch inerten Komponente hergestellt, sondern beide Komponeten werden zusammen und gleichzeitig miteinander vermahlen. Ein bestimmtes Verhältnis der Korngrößen der katalytisch aktiven und der katalytisch inerten Komponenten wird dabei nicht eingehalten. Aus der DE 43 08 120 A1 sowie der WO 94/15708 A1 sind nämlich keinerlei Angaben über dieses Korngrößenverhältnis zu entnehmen. Wird aber auf ein bestimmtes Korngrößenverhältnis der aktiven und inerten Komponente nicht geachtet, so treten die im folgenden genannten Nachteile auf, deren Ursachen vom Erfinder gefunden worden sind.

Die spezifische Oberfläche SA (in m$^2$/g) ultrafeiner Partikel im Größenbereich von 1000 mm und weniger hängt von der spezifischen Dichte D (in g/ml) und dem mittleren Teilchendurchmesser $d_{50}$ (in nm) ab und kann mit der allgemeinen Formel

$$SA = 6000/(D * d_{50}) \qquad (1)$$

abgeschätzt werden. Daraus ergeben sich bei $d_{50}$ = 500 nm folgende Werte für die spezifischen Oberflächen einiger typischer Katalysatorkomponenten:

| | |
|---|---|
| $TiO_2$ | 3,0 m$^2$/g |
| $CuO$ | 1,9 m$^2$/g |
| $Cr_2O_3$ | 2,3 m$^2$/g |
| $Al_2O_3$ | 3,0 m$^2$/g |
| $SiO_2$ | 5,5 m$^2$/g |
| $ZnO$ | 2,1 m$^2$/g |

Die Partikel der spezifisch leichteren inerten Komponenten haben daher eine größere spezifische Oberfläche als die Partikel der spezifisch schwereren aktiven Komponenten bei gleichen Korngrößen. Ein gemeinsames Vermahlen der aktiven und der inerten Komponenten auf eine gleiche Korngröße führt daher zu einer größeren spezifischen Oberfläche der inerten Partikel bezogen auf die aktiven Partikel. Eine Belegung der Oberfläche der aktiven Partikel mit den inerten Partikeln und damit eine Reduzierung der Katalysatoraktivität ist die Folge.

Neben diesem Nachteil zeichnet sich das aus den beiden zuletzt genannten Schriften bekannte Verfahren außerdem dadurch aus, daß während der Zerkleinerung eine sehr hohe Leistung auf die Ausgangsstoffe aufgebracht wird. So liegt in den Ausführungsbeispielen die bei der Vermahlung eingetragene Energie bei 30 bzw. 150 kW pro Liter Reaktorvolumen. In den bevorzugten Ausführungsformen des bekannten Verfahrens wird sogar von Energiedichten bis zu 500 kW pro Liter Reaktorvolumen gesprochen. Bei diesen Energieeinträgen handelt es sich nicht nur um eine mechanische Zerkleinerung, sondern, wie auch in der DE 43 08 120 A1 festgestellt wird, um eine Festkörperreaktion ähnlich wie im Fall des bereits oben genannten mechanischen Legierens (WO 90/09846). Vermahlungen mit derart hohen Energieeinträgen erfordern apparativ aufwendige Kühleinrichtungen. Darauf weist auch die bis zu 400 °C betragene Temperatur beim Zerkleinern der Ausgangsstoffe hin.

Beim Aufarbeitungsverfahren nach der DE 42 09 292 A1 werden die verbrauchten Katalysatoren ebenfalls mechanisch zerkleinert, bis eine Korngröße kleiner als 10 $\mu$m, vorzugsweise eine Korngröße zwischen 0,1 $\mu$m und 4 $\mu$m erreicht ist. Bei der Zerkleinerung beaufschlagt man den Katalysator mit einer mechanischen Energie einer Energiedichte bis zu 500 kW pro Liter Reaktorvolumen, um eine Festkörperreaktion durchzuführen. Der aufgearbeitete Katalysator läßt sich erneut zum Durchführen von Reaktionen einsetzen.

In diesem Verfahren werden die aktiven und die inerten Komponenten gemeinsam und damit auf eine gleiche Teilchengröße zerkleinert, so daß aus dem bereits oben genannten Grund eine verminderte Katalysatoraktivität resultiert.

Der Erfindung liegt die Aufgabe zugrunde, ein Verfahren zum Herstellen eines heterogenen Katalysators der eingangs genannten Art anzugeben, der die folgenden Anforderungen erfüllt. Auf wirtschaftliche Weise sollen Katalysatoren herstellbar sein, deren Komponenten in ihrer löslichen Form ein Löslichkeitsprodukt von mehr als $10^{-9}$ haben, zum Beispiel Titandioxid mit einem Löslichkeitsprodukt von etwa $10^{-5}$, und die aus diesem Grunde nicht auf dem Wege der Fällung der bzw. Mischfällung hergestellt werden können. Die bei der Fällung bzw. Mischfällung geltende Einschränkung, daß das Löslichkeitsprodukt der inerten Komponente geringer als das Löslichkeitsprodukt der katalytisch aktiven Komponente sein muß, soll im erfindungsgemäßen Herstellungsverfahren nicht gelten. Es sollen keine schwierig zu entsorgenden, nach der Herstellung des Katalysators zurückbleibenden, schwermetallhaltigen Rückstände entstehen. Das Herstellungsverfahren soll außerdem in kurzer Zeit und mit nur geringem apparativen Aufwand durchführbar sein. So soll die bei der Katalysatorherstellung durch mechanisches Legieren notwendige extrem starke Kühlung der Mühle bzw. des Attritors nicht erforderlich sein.

Diese Aufgabe wird heim Verfahren der eingangs genannten Art dadurch gelöst, daß man eine oder mehrere Suspensionen von Feststoffpartikeln der katalytisch aktiven Komponente/Komponenten (A) und eine oder mehrere Suspensionen von Feststoffpartikeln der katalytisch inerten Komponente/Komponenten (I) herstellt. Dabei ist der mittlere Korndurchmesser der inerten Komponenten größer als der mittlere Korndurchmesser der aktiven Komponente. Dann gibt man die Suspensionen unter Einwirkung von Ultraschall zusammen, trennt die Suspensionsflüssigkeit ab und unterwirft gegebenenfalls die erhaltenen Feststoffpartikel einer Formgebung. Vorzugsweise ist der mittlere Korndurchmesser der inerten Komponenten mindestens 1,1 mal, insbesondere mindestens 1,5 mal und besonders bevorzugt mindestens 3,5 mal größer als der mittlere Korndurchmesser der aktiven Komponente.

Im Gegensatz zu dem bekannten Verfahren nach der DE 43 08 120 A1 sind erfindungsgemäß keine zusätzlichen Suspendierhilfsmittel und Antistatika erforderlich, um die festen, ungelösten Teilchen in der Schwebe zu halten und um eine Reagglomeration der Partikel zu vermeiden. Derartige Hilfsmittel sind weder bei der Herstellung der Einzelkomponenten noch beim Zusammenmischen unter Ultraschallbedingungen notwendig. Typischerweise erhält man im erfindungsgemäßen Verfahren Suspensionen mit einer mittleren Dichte oberhalb von Wasser, die im wäßrigen Medium erst nach 15 bis 30 min sedimentieren.

Eine Reagglomeration der Teilchen des Gesamtkomponentensystems, also der aus der inerten und der aktiven Komponente zusammengesetzten feinen Teilchen, wird durch eine geeignete Wahl des Korngrößenverhältnisses von aktiver und inerter Komponente vermieden. Damit erreicht man eine starke Anziehung zwischen den kleinen und den großen Partikeln, nicht aber zwischen gleichgroßen zusammengesetzten oder nicht zusammengesetzten Teilchen. Charakteristisch für das erfindungsgemäße Verfahren sind daher einerseits die gute Filtrierbarkeit der Suspension der zusammengesetzten Teilchen und die hohen Trocknungsgeschwindigkeiten nach dem Abtrennen der Suspensionsflüssigkeit.

Die Möglichkeit, im erfindungsgemäßen Verfahren ohne Antistatika zu arbeiten, bietet besondere Vorteile, da derartige Stoffe in der Regel starke Katalysatorgifte sind.

Überraschenderweise wurde gefunden, daß es ausreicht, die Suspensionen der aktiven und inerten Komponenten mit dem genannten Korngrößenverhältnis unter Einwirkung von Ultraschall zusammenzugeben, um einen auch bei hoher mechanischer und thermischer Beanspruchung stabilen Katalysatorvorläufer unter reproduzierbaren Bedingungen herzustellen. Der eingesetzte Ultraschall dient zur gleichmäßigen Durchmischung und Homogenisierung der zusammengebrachten Suspensionen. Die aktiven und inerten Komponenten sind bei normaler mechanischer und thermischer Beanspruchung sowie bei Ultraschallbehandlung nicht mehr voneinander zu trennen. Das genannte Verhältnis der mittleren Korndurchmesser ermöglicht eine äußerst feine Verteilung der aktiven Katalysatorkomponente auf der inerten Komponente, da die feineren katalytisch aktiven Partikel sich fest an den größeren, katalytisch inerten Partikeln anlagern, aber eine Belegung der Oberfläche der aktiven Teilchen mit den inerten Teilchen nicht auftritt.

Zusammen mit der Wahl des Korngrößenverhältnisses von aktiver zu inerter Komponente ist der Einsatz von Ultraschall beim Zusammengeben der Suspensionen der Einzelkomponenten in dieser Erfindung besonders wichtig. Der Ultraschall wird hier zum Durchmischen der nanokristallinen Feststoffsuspensionen eingesetzt. Anders als beim Einsatz von klassischen Rührorganen, z. B. Propellerrührern oder Blattrührern, die nur größere Einheiten der Suspension durchmischen können, ermöglicht die Ultraschallbehandlung eine Durchmischung von Bereichen im Nanometerbereich. Werden die genannten Korngrößenverhältnisse von aktiver und inerter Komponente eingehalten, so erfolgt beim Einsatz des Ultraschalls keine Entmischung der zusammengegebenen Suspensionen. Liegt dagegen das Verhältnis der Korngrößen von inerter und aktiver Komponente deutlich über dem erfindungsgemäßen Bereich, so spaltet der Ultraschall die Suspension, und eine Phasentrennung tritt auf. Im entgegengesetzten Fall, bei einem unterhalb des erfindungsgemäßen Bereiches liegenden Korngrößenverhältnis von inerter und aktiver Komponente lagern sich die zu kleinen inerten Partikel auf dem relativ großen aktiven Teilchen ab, so daß der Katalysator blockiert und seine Aktivität reduziert ist. Dieser Fall tritt bei einer gleichzeitigen Zerkleinerung von inerter und aktiver Komponente auf eine gleiche mittlere Korngröße auf, wie bereits oben ausgeführt worden ist.

Im Falle des erfindungsgemäßen Verhältnisses der Korngrößen von inerter und aktiver Komponente dagegen verteilt der Ultraschall die aktive Komponente in hinsichtlich der Katalysatoraktivität optimaler Weise auf die größeren inerten Partikel.

Typisch für den hergestellten Katalysator ist das Fehlen von üblichen Lösungsmitteln und von löslichen Bestandteilen, zum Beispiel von Nitratresten. Derartige Bestandteile liegen unterhalb der Nachweisgrenze, z. B. unter 1 ppm. Charakteristisch ist ferner die im Elektronenmikroskop sichtbare runde Gestalt der Primärpartikel, die sich erfindungsgemäß aneinander angelagert haben. Ein weiteres Kennzeichen des erfindungsgemäßen Katalysators ist die Existenz von katalytisch inerten Komponenten mit einem im Vergleich zum Verfahren nach der Mischfällung relativ hohen Löslichkeitsprodukt, zum Beispiel Titanoxidhydrat oder Siliciumoxidhydrat. Die katalytisch aktiven und inerten Komponenten sind ferner nicht miteinander legiert wie im Falle der aus den oben genannten Druckschriften bekannten Katalysatoren. Vielmehr handelt es sich um eine Art Adduktbildung.

Mit dem erfindungsgemäßen Herstellungsverfahren werden eine Reihe von Vorteilen erreicht. Im Gegensatz zum Herstellen mittels Fällung bzw. Mischfällung fallen nach der Herstellung keine Flüssigkeiten an, die aufwendig entsorgt werden müßten. Nach der Abtrennung des Katalysatorvorläufers aus der Suspensionsflüssigkeit läßt sich diese Suspensionsflüssigkeit nämlich zur erneuten Herstellung von Ausgangssuspensionen wieder verwenden. Eine solche Kreislaufführung ist bei dem Herstellungsverfahren durch Fällung bzw. Mischfällung nach dem Stand der Technik nicht möglich, da das Lösungsmittel in großen Mengen die Neutralsalze der zur Ausfällung aufgelosten Stoffe enthält. Eine wie auch immer geartete Aufarbeitung durch Eliminierung der Neutralsalze, z. B. Eindampfen oder Destillieren, liegt außerhalb jeder Wirtschaftlichkeit.

Das erfindungsgemäße Herstellungsverfahren läßt sich mit nur geringem apparativen Aufwand durchführen. So können die Ausgangssuspensionen durch Vermahlen der aktiven bzw. inerten Komponente in Gegenwart der Suspensionsflüssigkeit durchgeführt werden, wobei die Suspensionsflüssigkeit gleichzeitig zum größten Teil als Kühlmittel dient. Besondere Kühlmittel wie beim mechanischen Legieren oder bei anderen, oben genannten Festkörperreaktionen sind hier nicht notwendig, da mit weitaus geringeren Energieeinträgen gearbeitet werden kann.

Die aktiven und inerten Komponenten der Suspensionen haben bevorzugt Korngrößen bis zu 1000 nm und insbesondere bis zu 800 nm.

Vorzugsweise setzt man als Suspensionsflüssigkeit Wasser ein.

Die bereits oben genannte besonders umweltfreundliche Verfahrensführung erreicht man, wenn man nach dem Abtrennen der Feststoffpartikel die Suspensionsflüssigkeit zum Herstellen von Suspensionen der katalytisch aktiven und/oder inerten Komponenten zurückführt. Vorzugsweise wird die Suspensionsflüssigkeit durch Sedimentation von den hergestellten Massivkatalysatoren abgetrennt. In der Flüssigkeit verbleiben damit nur wenige kolloidale Reste von Feststoffen. Diese Reststoffe stören beim erneuten Einsatz der Suspensionsflüssigkeit nicht. Nur im Falle eines Produktwechsels sollten auch die kolloidalen Bestandteile abgeschieden werden. Man trennt diese Bestandteile vorzugsweise mittels Ultrafiltration ab.

Die Suspensionsflüssigkeit kann auch gelöste Bestandteile enthalten, die von eingesetzten frischen Rohstoffen stammen. Typischerweise sind in kommerziell erhältlichem Titandioxid und Kupferoxid geringe Anteile der Katalysatorgifte Kupfersulfat und Titanylsulfat/Titansulfat enthalten. Ein besonderer Vorteil des erfindungsgemäßen Verfahrens liegt in der verstärkten Lösung dieser Anteile durch die Einwirkung des Ultraschalls und in der Abtrennung der gelösten Bestandteile gemeinsam mit der Suspensionsflüssigkeit, bevor die Partikel einer Formgebung unterworfen werden. Damit wird eine besonders hohe Katalysatorqualität erreicht. Eine solche Reinigung des Ausgangsmaterials ist beim Rohstoffhersteller in der Regel nicht oder nur mit einem unzumutbar hohen wirtschaftlichen Aufwand möglich. Wenn sich beim erfindungsgemäßen Verfahren die gelösten Rohstoffe im Kreislaufwasser stärker angereichert haben, können sie in Reinigungsstufen abgetrennt werden, die in den Kreislauf geschaltet sind. Ein Beispiel für solche Reinigungsstufen findet sich im weiter unten erläuterten Ausführungsbeispiel.

In einer wirtschaftlichen Ausgestaltung der Erfindung stellt man die Suspension von Feststoffpartikeln der katalytisch aktiven und/oder inerten Komponenten durch Vermahlen in Anwesenheit der Suspensionsflüssigkeit her. Die Suspensionsflüssigkeit, insbesondere Wasser, dient dabei zum Verteilen und Abführen der beim Vermahlen entstehenden Wärme.

Bei der Herstellung der feinteiligen Komponenten hat es sich als vorteilhaft herausgestellt, wenn man die Komponenten in einer Reibmühle, insbesondere in einer Ringspaltmühle, vermahlt. Bei der Ringspaltmühle dreht sich ein zentrisch gelagerter Mahlkegel in einem glockenförmigen Hohlkegel. Das zu vermahlende Gut tritt unten in die Mühle ein, wird im Ringspalt zwischen dem Mahlkegel und der Innenwand des Gehäuses zerkleinert und tritt im Oberteil der Mühle, die auch Glockenmühle genannt wird, aus. Ein besonderer Vorteil dieser Mühle ist das sehr enge Kornspektrum des erhaltenen Produkts. Es liegt aber auch im Rahmen der Erfindung, andere Mühlen oder andere Zerkleinerungsapparate zum Herstellen der für die Herstellung des Katalysators vorgesehenen Pulver bzw. Ausgangssuspensionen einzusetzen.

Zur Verstärkung der Mahlwirkung können in der Reibmühle zusätzlich Mahlkugeln eingesetzt werden.

Zur Herstellung der Ausgangssuspensionen bzw. der feinteiligen Pulver wird vorgeschlagen, daß die Mahlleistung bei höchstens 10 kW/l Mahlgutvolumen, vorzugsweise bei höchstens 3 kW/l liegt. Bei diesem Energieeintrag ist eine in die Mühle integrierte Kühleinrichtung nicht notwendig, da die schnell durch die Mühle hindurchlaufende Suspensionsflüssigkeit die Wärme mit sich führt, so daß eine externe Kühlung der umlaufenden Suspensionsflüssigkeit ausreicht. Vorzugsweise wird das Wasser in sämtlichen Kreislaufabschnitten (Ultraschallbehandlung, Sedimentation, Ultrafiltration, usw.) auf Raumtemperatur gehalten. Zusätzlich kann ein interner Kühlwasserkreislauf in der Mühle zur Beseitigung geringfügiger Temperaturgradienten vorgesehen sein.

Beim Zusammengeben der Suspensionen der feinteiligen Komponenten kann man im Rahmen der Erfindung auf unterschiedliche Weise vorgehen. Vorzugsweise gibt man die Suspensionen der feinteiligen Komponenten unter Rühren zusammen, wobei die eingetragene Rührleistung insbesondere bei mindestens 0,4 kW/l liegt.

Weiterhin ist es außerdem von Vorteil, wenn der beim Zusammengeben der Suspensionen der feinteiligen Komponenten einwirkende Ultraschall eine Leistung bis 4000 W/l, insbesondere von 200 bis 600 W/l, eine Amplitude von 100 bis 300 $\mu$m und eine Frequenz von 16 bis 30 kHz hat.

Nach dem Abtrennen der Suspensionsflüssigkeit trocknet und tablettiert oder extrudiert man die erhaltenen Feststoffpartikel, gegebenenfalls unter Zusatz eines Plastifizierungsmittels, zum Beispiel organischer Ester und/oder langkettiger Alkohole.

Vorgeschlagen wird außerdem, daß man die Suspension einer Sprühtrocknung und insbesondere anschließend die erhaltenen Granulate einer thermischen Zersetzung noch vor der Formgebung unterwirft. Die Sprühtrocknung als eine Trennung zwischen fest und flüssig nimmt man insbesondere nach einem Abtrennen des größten Teils der Suspensionsflüssigkeit in einem Absetzer vor. Die anschließende thermische Zersetzung ist insbesondere dann vorteilhaft, wenn man als Ausgangskomponenten Hydroxide und/oder Carbonate der entsprechenden Metalle verwendet, die zu ihren Oxiden thermisch zersetzt werden. Bei der Formgebung kann es sich insbesondere um eine Extrusion handeln.

Das erfindungsgemäße Verfahren läßt sich zum Herstellen einer Vielzahl unterschiedlicher Katalysatoren durchführen. In einer bevorzugten Ausgestaltung der Erfindung setzt man als katalytisch aktive Komponente Kupferoxid (CuO) und als inerte Komponente Titandioxid ein. Dieser Katalysator läßt sich mit Vorteil zum Hydrieren von nativen Fetten, Ölen und Fettderivaten, zum Beispiel Fettsäuremethylester verwenden. Ein derartiger Katalysator hat besondere Vorteile, da die inerte Komponente Titandioxid zum einen preiswert ist und zum anderen sich aufgrund ihrer Reaktionsträgheit besonders gut als Dispergierkomponente im Katalysator eignet.

Allgemein wird vorgeschlagen, daß man als katalytisch aktive Komponente Kupferoxid (CuO), metallisches Kupfer, metallisches Nickel, eine Kupfer-Nickel-Legierung und/oder Oxide und/oder Mischoxide von Nickel, Kobalt, Vanadium, Molybdän, Eisen, Zinn, Silber, Chrom, Yttrium, Barium, Lanthan, Strontium, Wismut und/oder Zink und/oder metallisches Platin, metallisches Palladium und/oder metallisches Rhenium oder eine Mischung oder Legierung/Verbindung daraus einsetzt.

Als inerte Komponenten eignen sich insbesondere Titandioxid, Zinkoxid, Siliciumdioxid, Zirkondioxid, Zirkonsilicat und/oder Aluminiumoxid oder eine Mischung/Verbindung daraus.

Für die Aktiv- und Inertkomponenten lassen sich nicht nur Metalloxide, sondern auch Hydroxide und Carbonate einsetzen, die in einer nachgeschalteten Calzinierstufe thermisch zu den entsprechenden Metalloxiden zersetzt werden. Da ganz allgemein wasserunlösliche, aber thermisch zu den Oxiden zersetzbare Verbindungen dieser Metalle im erfindungsgemäßen Verfahren mit Vorteil verwendet werden können, wird vorgeschlagen, daß man als katalytisch aktive und/oder inerte Komponenten wasserunlösliche, zu ihren Metalloxiden thermisch zersetzbare Verbindungen einsetzt, wobei Hydroxide und Carbonate bevorzugt sind.

Durch das erfindungsgemäße Verfahren wird ein heterogener, massiver Katalysator hergestellt der aus mindestens einer katalytisch aktiven (A) und mindestens einer katalytisch inerten (I) Komponente besteht, wobei die Komponenten ineinander dispergiert sind.

Die bereits oben genannte erfindungsgemäße Aufgabe wird bei diesem Katalysator dadurch gelöst, daß der Katalysator gekennzeichnet ist durch Partikel aus der katalytisch inerten Komponente, auf denen Partikel aus der katalytisch aktiven Komponente aufgewachsen sind, wobei der mittlere Korndurchmesser der inerten Partikel größer als der mittlere Korndurchmesser der aktiven Komponente ist. Vorzugsweise haben die aktiven und die inerten Partikel einen mittleren Korndurchmesser bis zu 1100 nm, insbesondere bis zu 800 nm. Bevorzugt ist außerdem, daß der mittlere Korndurchmesser der inerten Partikel mindestens 1,1 mal, vorzugsweise mindestens 1,5 mal und besonders bevorzugt mindestens 3,5 mal größer als der mittlere Korndurchmesser der aktiven Komponente ist. Die separaten aktiven oder inerten Partikel werden im folgenden als Vorstufe des Katalysators bezeichnet, der aus den inerten Partikel mit aufgewachsenen aktiven Partikeln besteht.

In einer besonders bevorzugten Ausführungsform des hergestellten Katalysators besteht die Vorstufe der katalytisch aktiven Komponente aus Kupferoxid (CuO) mit einem mittleren Korndurchmesser von 200 bis 500 nm und die inerte Komponente aus Titandioxid mit einem mittleren Korndurchmesser von 600 bis 1000 nm besteht.

Weiterhin wird vorgeschlagen, daß die Vorstufe der katalytisch aktiven Komponente des Katalysators Kupferoxid (CuO), metallisches Nickel, metallisches Kupfer, eine Kupfer-Nickel-Legierung und/oder Oxide und/oder Mischoxide von Nickel, Kobalt, Vanadium, Molybdän, Eisen, Zinn, Silber, Chrom, Yttrium, Barium, Lanthan, Strontium, Wismut und/oder von Zink und/oder metallisches Platin, metallisches Palladium und/oder metallisches Rhenium oder eine Mischung oder Legierung/Verbindung daraus ist. Diese Komponenten sind zum Teil Hauptbestandteile, zum Teil - z. B. im Falle von Strontium, Wismut, Barium - Promotoren.

Als inerte Komponente des Katalysators wird Titandioxid, Zinkoxid, Siliciumdioxid, Zirkondioxid, Zirkonsilicat und/oder Aluminiumdioxid oder eine Mischung/Verbindung daraus vorgeschlagen.

Als Aktiv- und Inertkomponente lassen sich nicht nur Metalloxide verwenden. Es wird außerdem vorgeschlagen, daß die katalytisch aktive und/oder die inerte Komponente mindestens eine wasserunlösliche und thermisch zu ihren Metalloxiden zersetzbare Verbindung, insbesondere aus der Gruppe der Hydroxide und/oder Carbonate, ist.

Die Katalysatoren eignen sich je nach Zusammensetzung zum Durchführen einer Vielzahl von Reaktionen. Insbesondere wird die Verwendung eines derartigen erfindungsgemäßen Katalysators zum Hydrieren, Härten oder Oxidieren, insbesondere zum Hydrieren und Härten nativer Öle, Fette, Fettsäuren, Fettsäurealkylester und anderer Fettsäurederivate vorgeschlagen. Zum Hydrieren von Fettsäurealkylestern eignen sich beispielsweise kupferhaltige Katalysatoren. Nickelhaltige Katalysatoren sind besonders gut zum Härten nativer Öle, Fette und Fettsäuren geeignet.

Die Erfindung und Ausführungsbeispiele dazu sind nachfolgend anhand von Zeichnungen näher erläutert. Es zeigen

Figur 1    eine schematische Darstellung der Ausgangskomponenten sowie des fertigen Katalysators mit den Korngrößenverteilungen,

Figur 2    ein Fließbild des erfindungsgemäßen Herstellungsverfahrens nach einem Ausführungsbeispiel,

Figur 2a    ein abgewandeltes Fließbild des Verfahrens nach Figur 2 und

Figur 3    die Aktivitäten verschiedener erfindungsgemäßer Katalysatoren sowie eines Katalysators nach dem Stand der Technik, dargestellt in einem Diagramm.

In allen Zeichnungen haben gleiche Bezugszeichen die gleiche Bedeutung und werden daher gegebenenfalls nur einmal erläutert.

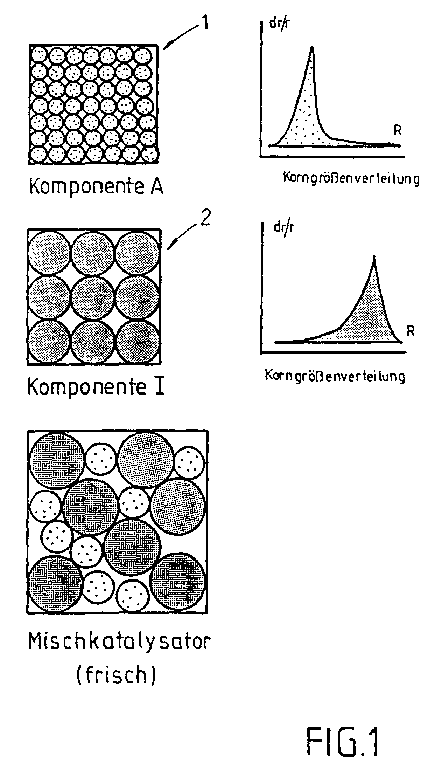

Teilbild 1 in Figur 1 zeigt schematisch die feinteiligen Partikel der katalytisch aktiven Ausgangskomponente A. In entsprechender Weise zeigt Teilbild 2 die katalytisch inerte Komponente I. Rechts neben den Teilbildern 1 und 2 sind die Korngrößenverteilungen dr/r, aufgetragen über dem Radius, abgebildet. Neben der deutlich höheren mittleren Korngröße der Komponente I wird auch deutlich, daß sich die Korngrößenverteilungen der beiden Komponente A und I in einem nur geringen Umfang überlappen. Da diese schematischen Diagramme nur zur qualitativen Erläuterung des erfindungsgemäßen Konzeptes dienen sollen, tragen die Achsen keine Bemessungen.

Im unteren Teil der Figur 1 ist schematisch ein Querschnitt durch den fertigen erfindungsgemäßen Katalysator gezeigt. Man erkennt die kleineren Partikel der katalytisch aktiven Komponente A, die zwischen den größeren Partikeln der katalytisch inaktiven Komponente I angeordnet sind.

Die Diagramme in Figur 1 beziehen sich auf einen aus frischen Rohstoffen hergestellten Katalysator, also nicht auf einen aufgearbeiteten Katalysator.

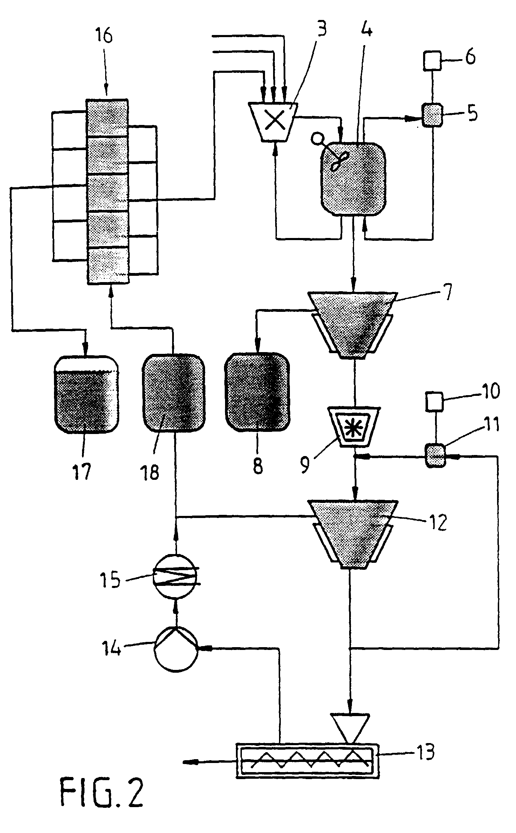

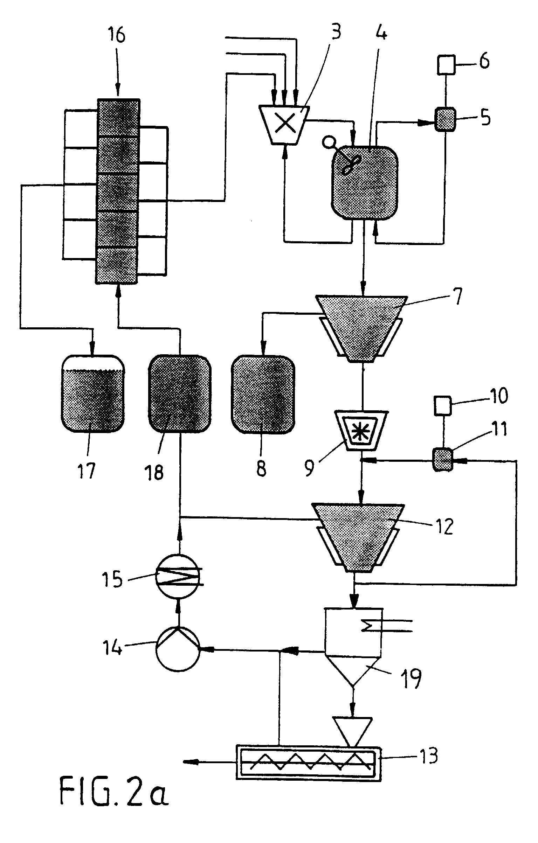

Im Fließbild von Figur 2 werden die frischen Rohstoffe, gegebenenfalls zusammen mit Sekundärrohstoffen aus der Aufarbeitung verbrauchter Katalysatoren, mit rückgeführter Suspensionsflüssigkeit in einer Mühle 3 vorzerkleinert und in einem Rührkessel 4 aufgeschlämmt. Bei den verbrauchten Katalysatoren handelt es sich in diesem Beispiel um zum Hydrieren ("Härten") nativer Öle eingesetzte Katalysatoren. Zum Homogenisieren der Suspension wird ein Teil der Suspension laufend aus dem Rührkessel 4 herausgeführt und in einem Behälter 5 mit Ultraschall einer ersten Ultraschallquelle 6 behandelt. Die Vorzerkleinerung und die Homogenisierung werden im Kreislauf durchgeführt, wie es im oberen Teil der Figur 2 dargestellt ist.

Nach einer ersten Absetzstufe 7, in der das an den verbrauchten Katalysatoren anhaftende Fett abgetrennt und in einen Behälter 8 abgeleitet wird, wird die homogenisierte Suspension in einer Ringspaltmühle 9 auf eine Korngröße unter 1100 nm vermahlen.

In einer zweiten Absetzstufe sedimentiert die Suspension, und die Trägerflüssigkeit wird abgetrennt und wieder zurückgeführt. Anschließend homogenisiert man die auf diese Weise erhaltene äußerst feinkörnige Suspension durch Einwirkung einer zweiten Ultraschallquelle 10 auf den durch den Behälter 11 geführten, im Kreis gefahrenen Teilstrom. Der Absetzbehälter 12 dient außerdem zur Stabilisierung der Suspension und als Pufferbehälter für den nachfolgenden Vakuumextruder 13, in dem die erhaltene Katalysatormasse getrocknet, verdichtet und durch Lochdüsen gepreßt wird. Die erhaltenen Stränge werden in an sich bekannter Weise in Granulate zerteilt.

Die mit der Vakuumpumpe 14 abgesaugte feuchte Luft wird im Wärmetauscher 15 gekühlt. Die kondensierte Flüssigkeit wird zusammen mit der aus dem Absetzbehälter 12 abgetrennten Flüssigkeit in einem Behälter 18 gesammelt und in einer Ultrafiltrationsanlage 16 von kolloidalen Partikeln befreit, die in den Behälter 17 geleitet werden, wo sie sedimentieren. Die Ultrafiltration ist nur bei einem Produktwechsel notwendig, sofern ausschließlich frische Katalysatorrohstoffe und keine Sekundärrohstoffe aus verbrauchten Katalysatoren eingesetzt werden.

Die gereinigte Suspensionsflüssigkeit wird schließlich zur Vorzerkleinerungsstufe zurückgeführt.

Das Fließbild nach Figur 2a zeigt eine Abwandlung des Verfahrens nach Figur 2. Hier wird zusätzlich ein Sprühtrockner 19 verwendet, in dem auch eine thermische Behandlung und Granulation stattfindet. Das bei der Sprühtrocknung entzogene Wasser wird direkt zur Pumpe 14 geführt. Erst im Anschluß an die Sprühtrocknung, die thermische Behandlung und Granulation findet die Formgebung im Extruder 13 statt. Die thermische Behandlung ist insbesondere wichtig, wenn als Rohstoffe Hydroxide, Carbonate und andere, zu den entsprechenden Metalloxiden thermisch zersetzbare Metallverbindungen eingesetzt werden. Alternativ kann die thermische Behandlung, die Calzinierung, auch in einem in Figur 2a nicht dargestellten, zwischen dem Apparat 19 und dem Extruder 13 geschalteten separaten Apparat vorgenommen werden.

Beispiel 1

1 kg pulverförmiges Kupferoxid (Fa. Riedel de Haen, mittlere Korngröße 100 $\mu$m) wird unter Rühren bei einem Energieeintrag von 0,5 kW in 2 kg destilliertem Wasser aufgeschlämmt. Zum Homogenisieren der Suspension wird diese über die erste Ultraschallstufe 5 (Leistung 400 W/l, Amplitude 150 $\mu$m bei 24 kHz) mit einer Durchflußrate von 50 l/h geführt. Die so homogenisierte Suspension wird anschließend in einer Ringspaltmühle 9 (Typ MS-12 der Fa. Fryma AG) bei einem Energieeintrag von 2,5 kW/l auf eine Kornfeinheit von 400 nm zerkleinert und dann im Durchfluß durch die zweite Ultraschallstufe 11 homogenisiert (Kreislauffahrweise über zweite Absetzstufe, Pufferbehälter 12).

Als katalytisch inerte Komponente wird 1,3 kg feinkörniger Quarz (Fa. Merck, Korngrößenfraktion < 200 $\mu$m) aufgeschlämmt, homogenisiert und in der Ringspaltmühle 9 mit 2,5 kW/l auf einen $d_{50}$-Wert von 1140 nm zerkleinert und der Kupferoxidsuspension zugegeben.

Das Suspensionsgemisch wird in der zweiten Ultraschallstufe 11 (Einstellung 400 W/l, Amplitude 200 $\mu$m bei 24 kHz) im zweimaligen Durchlauf homogenisiert. Man erhält eine homogene Suspension mit einem Durchmesser- bzw. Dichteverhältnis

$$D(SiO_2)/D(CuO) = d(CuO)/d(SiO_2) = 0,35.$$

Das Trägerfluid wird nach einer sedimentativen Abtrennung wieder in den Prozeß zurückgeführt. Nach der Eindickung der Suspension auf etwa 15 bis 20 Gew.-% in der zweiten Absetzstufe 12 wird die Katalysatormasse im Vakuumextruder 13 (Fa. Händle, Typ XC) bei 10 bis 20 mbar und einem Temperaturbereich von 110 bis 130 °C getrocknet und zu Granulaten mit Durchmessern von 3 mm geformt.

Beispiel 2

1 kg pulverförmiges Kupferoxid (Fa. Riedel de Haen, mittlere Korngröße 100 $\mu$m) wird wie im Beispiel 1 in 2 kg destilliertem Wasser aufgeschlämmt, homogenisiert und auf eine mittlere Kornfeinheit von 400 nm zerkleinert.

Als katalytisch inerte Komponente wird 1 kg pulverförmiges Titandioxid (Typ 3000 der Fa. Kronos Titan GmbH, mittlere Korngröße 400 $\mu$m) auf die oben beschriebene Weise in 2 kg destilliertem Wasser suspendiert, homogenisiert, mit 1,5 kW/l auf eine mittlere Korngröße von 640 nm in der Ringspaltmühle 9 zerkleinert und zur Kupferoxidsuspension zugegeben.

Das Suspensionsgemisch wird in der zweiten Ultraschallstufe 11 (Einstellung wie oben) im zweimaligen Durchlauf homogenisiert. Man erhält eine homogene Suspension mit einem Durchmesser- bzw. Dichteverhältnis

$$D(TiO_2)/D(CuO) = d(CuO)/d(TiO_2) = 0,63,$$

die sich als homogene Phase leicht von der Flüssigkeit trennt.

Das Trägerfluid wird nach der Abtrennung wieder in den Prozeß zurückgeführt. Nach der Eindickung der Suspension auf etwa 15 bis 20 Gew.-% Feststoff in der Zweiten Absetzstufe 12 wird die Katalysatormasse im Vakuumextruder 13 (Typ XC der Fa. Händle) bei 10 bis 20 mbar im Temperaturbereich von 110 bis 130 °C getrocknet und zu Granulaten mit Durchmessern von 3 mm geformt.

Beispiel 3

1 kg pulverförmiges Kupferoxid (Fa. Riedel de Haen, mittlere Korngröße 100 µm) wird wie in den Beispielen 1 und 2 in 2 kg destilliertem Wasser aufgeschlämmt, homogenisiert und auf eine mittlere Kornfeinheit von 400 nm zerkleinert.

Als katalytisch inerte Komponente wird 1,01 kg Zinkoxid (Fa. Riedel de Haen, mittlere Korngröße 60 µm) in der Ringspaltmühle 9 mit 2,0 kW/l auf eine mittlere Korngröße von 450 nm zerkleinert und der Kupferoxidsuspension zugegeben.

Das Suspensionsgemisch wird in der zweiten Ultraschallstufe 11 (Einstellungen 400 W/l, Amplitude 200 µm bei 24 kHz) im zweimaligen Ultraschall-Durchgang homogenisiert. Man erhält eine homogene Suspension mit einem Durchmesser- bzw. Dichteverhältnis

$$D(ZnO)/D(CuO) = d(CuO)/d(ZnO) = 0,89.$$

Die Eindickung, Trocknung und Formgebung erfolgt entsprechend den Bedingungen in den Beispielen 1 und 2.

Aktivitätstests

Die gemäß den Beispielen 1 bis 3 hergestellten Katalysatoren wurden dem Standardtest bekannter Anwender von Katalysatoren für die Hydrierung von Fettsäuremethylester zu den entsprechenden Fettalkoholen (Firmen Henkel KGaA, Condea, Kao) unterworfen, um die Hydrieraktivität und Selektivität bei der Herstellung von Laurylalkohol durch Hydrieren von Laurinsäuremethylester zu bestimmen.

Testbedingungen:

Reaktor      1 l Rührautoklav
Druck        300 bar Wasserstoff
Temperatur   220 °C
Drehzahl     1000 $min^{-1}$
Einwaage     300 g Laurinsäuremethylester
             15 g Katalysatorpulver

Testergebnisse:

| Beispiel Nr. | Katalysatortyp | Umsatz zu Laurol (%) | Nebenprodukte (%) |
|---|---|---|---|
| 1 | $CuO/SiO_2$ | 81 | 1,7 |
| 2 | $CuO/TiO_2$ | 90 | 0,4 |
| 3 | CuO/ZnO | 83 | 0,7 |
| Vergleich | Kupferchromit | 87 | 0,9 |

Bei dem Vergleichskatalysator handelte es sich um einen kommerziellen Katalysator der Fa. Südchemie, der einer der besten im Handel erhältlichen Katalysatoren für die Hydrierung von Fettsäuremethylester zu Fettalkoholen darstellt.

Die Ergebnisse zeigen, daß der erfindungsgemäß hergestellte Katalysator zumindest etwa die gleiche Aktivität und Selektivität wie ein sehr guter Handelskatalysator zeigt. Im Gegensatz zu diesem läßt der erfindungsgemäße Katalysator sich jedoch auf eine erheblich wirtschaftlichere und umweltschonendere Weise herstellen.

Im Falle des $CuO/TiO_2$-Katalysators liegen sogar sowohl die Aktivität als auch die Selektivität deutlich über den Werten des Handels-Katalysators.

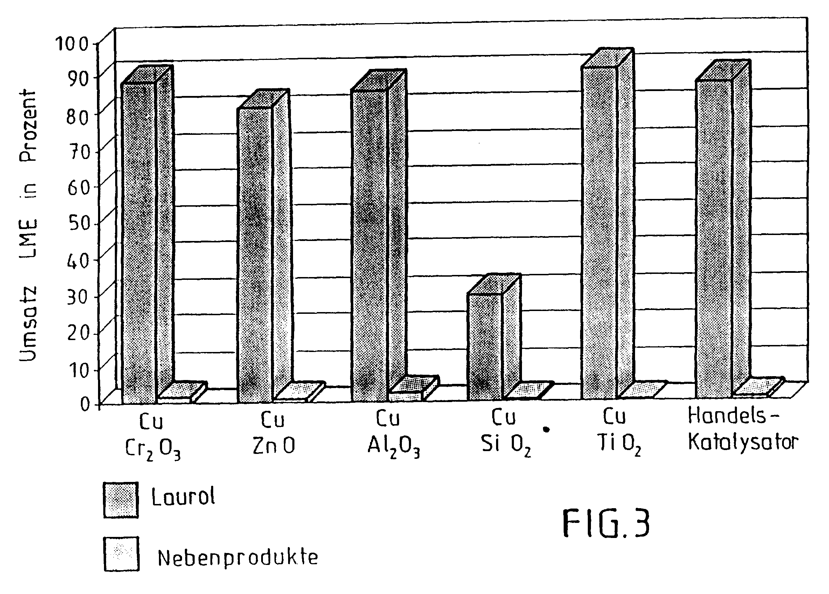

In Figur 3 sind die Ergebnisse von Aktivitätstests mit einigen erfindungsgemäßen Katalysatoren sowie dem vorgenannten handelsüblichen Katalysator (Kupferchromit) zusammengefaßt. Deutlich erkennbar ist die hohe Qualität der erfindungsgemäß hergestellten Katalysatoren.

**Bezugszeichenliste**

| 1 | Teilbild |
|---|---|
| 2 | Teilbild |
| 3 | Mühle |
| 4 | Rührkessel |
| 5 | Behälter |
| 6 | Ultraschallquelle |
| 7 | Absetzstufe |
| 8 | Behälter |
| 9 | Ringspaltmühle |
| 10 | Ultraschallquelle |
| 11 | Behälter |
| 12 | Absetzbehälter |
| 13 | Vakuumextruder |
| 14 | Vakuumpumpe |
| 15 | Wärmetauscher |
| 16 | Ultrafiltrationsanlage |
| 17 | Behälter |
| 18 | Behälter |
| 19 | Sprühtrockner |

**Patentansprüche**

1. Verfahren zum Herstellen eines heterogenen, massiven Katalysators, der aus mindestens einer katalytisch aktiven (A) und mindestens einer katalytisch inerten (I) Komponente besteht, wobei die Komponenten (A, I) ineinander dispergiert sind,
**dadurch gekennzeichnet**,
daß man eine oder mehrere Suspensionen von Feststoffpartikeln der katalytisch aktiven Komponente/Komponenten (A) und eine oder mehrere Suspensionen von Festoffpartikeln der katalytisch inerten Komponente/Komponenten (I) herstellt, wobei der mittlere Korndurchmesser der inerten Komponenten größer als der mittlere Korndurchmesser der aktiven Komponente ist, dann die Suspensionen unter Einwirkung von Ultraschall zusammengibt, die Suspensionsflüssigkeit abtrennt und gegebenenfalls die erhaltenen Feststoffpartikel einer Formgebung unterwirft.

2. Verfahren nach dem vorhergehenden Anspruch,
**dadurch gekennzeichnet**,
daß der mittlere Korndurchmesser der inerten Komponenten mindestens 1,1 mal, insbesondere mindestens 1,5 mal und besonders bevorzugt mindestens 3,5 mal größer als der mittlere Korndurchmesser der aktiven Komponente ist.

3. Verfahren nach einem der vorhergehenden Ansprüche,
**dadurch gekennzeichnet**,
daß die Komponenten (A, I) Korngrößen bis zu 1100 nm und insbesondere bis zu 800 nm haben.

4. Verfahren nach einem der vorhergehenden Ansprüche,
**dadurch gekennzeichnet**,
daß man als Suspensionsflüssigkeit Wasser einsetzt.

5. Verfahren nach einem der vorhergehenden Ansprüche,
**dadurch gekennzeichnet**,
daß man nach dem Abtrennen der Feststoffpartikel die Suspensionsflüssigkeit zum Herstellen von Suspensionen der katalytisch aktiven und/oder inerten Komponenten zurückführt.

**6.** Verfahren nach einem der vorhergehenden Ansprüche,
**dadurch gekennzeichnet**,
daß man die Suspensionen von Feststoffpartikeln der katalytisch aktiven und/oder inerten Komponenten durch Vermahlen in Anwesenheit der Suspensionsflüssigkeit herstellt.

**7.** Verfahren nach dem vorhergehenden Anspruch,
**dadurch gekennzeichnet**,
daß man die Komponenten separat in einer Reibmühle, insbesondere in einer Ringspaltmühle (9) vermahlt.

**8.** Verfahren nach dem vorhergehenden Anspruch,
**dadurch gekennzeichnet**,
daß zusätzlich Mahlkugeln eingesetzt werden.

**9.** Verfahren nach Anspruch 6 oder 7,
**dadurch gekennzeichnet**,
daß die Mahlleistung bei höchstens 10 kW/l Mahlgutvolumen, vorzugsweise bei höchstens 3 kW/l liegt.

**10.** Verfahren nach einem der vorhergehenden Ansprüche,
**dadurch gekennzeichnet**,
daß man die Suspensionen der feinteiligen Komponenten unter Rühren zusammengibt, wobei die eingetragene Rührleistung insbesondere bei mindestens 0,4 kW/l liegt.

**11.** Verfahren nach einem der vorhergehenden Ansprüche,
**dadurch gekennzeichnet**,
daß der beim Zusammengeben der Suspensionen der feinteiligen Komponenten einwirkende Ultraschall eine Leistung bis 4000 W/l, insbesondere von 200 bis 600 W/l, eine Amplitude von 100 bis 300 $\mu$m und eine Frequenz von 16 bis 30 kHz hat.

**12.** Verfahren nach einem der vorhergehenden Ansprüche,
**dadurch gekennzeichnet**,
daß man nach dem Abtrennen der Suspensionsflüssigkeit die erhaltenen Feststoffpartikel trocknet und tablettiert oder extrudiert, gegebenenfalls unter Zusatz eines Plastifizierungsmittels.

**13.** Verfahren nach einem der vorhergehenden Ansprüche,
**dadurch gekennzeichnet**,
daß man die Suspension einer Sprühtrocknung und insbesondere anschließend die erhaltenen Granulate einer thermischen Zersetzung noch vor der Formgebung unterwirft.

**14.** Verfahren nach einem der vorhergehenden Ansprüche,
**dadurch gekennzeichnet**,
daß man als katalytisch aktive Komponente Kupferoxid (CuO) und als inerte Komponente Titandioxid einsetzt.

**15.** Verfahren nach Anspruch 14,
**dadurch gekennzeichnet**,
daß die Vorstufe der katalytisch aktiven Komponente aus Kupferoxid (CuO) mit einem mittleren Korndurchmesser von 200 bis 500 nm und die inerte Komponente aus Titandioxid mit einem mittleren Korndurchmesser von 600 bis 1000 nm besteht.

**16.** Verfahren nach einem der vorhergehenden Ansprüche,
**dadurch gekennzeichnet**,
daß man als katalytisch aktive Komponente Kupferoxid (CuO), metallisches Kupfer, metallisches Nickel, eine Kupfer-Nickel-Legierung und/oder Oxide und/oder Mischoxide von Nickel, Kobalt, Vanadium, Molybdän, Eisen, Zinn, Silber, Chrom, Yttrium, Barium, Lanthan, Strontium, Wismut und/oder Zink und/oder metallisches Platin, metallisches Palladium und/oder metallisches Rhenium oder eine Mischung oder Legierung/Verbindung daraus einsetzt.

**17.** Verfahren nach einem der vorhergehenden Ansprüche,
**dadurch gekennzeichnet**,
daß man als inerte Komponente Titandioxid, Zinkoxid, Siliciumdioxid, Zirkondioxid, Zirkonsilicat und/oder Aluminiumoxid oder eine Mischung/Verbindung daraus einsetzt.

18. Verfahren nach einem der vorhergehenden Ansprüche,
**dadurch gekennzeichnet**,
daß man als katalytisch aktive und/oder inerte Komponenten wasserunlösliche, zu ihren Metalloxiden thermisch zersetzbare Verbindungen einsetzt, wobei Hydroxide und Carbonate bevorzugt sind.

**Claims**

1. A process for the production of a heterogeneous solid catalyst which consists of at least one catalytically active component (A) and at least one catalytically inert component (I), component (A) and component (I) being dispersed in one another, characterized in that one or more suspensions of solid particles of the catalytically active component(s) (A) and one or more suspensions of solid particles of the catalytically inert component(s) (I) are prepared, the mean particle diameter of the inert components being greater than the mean particle diameter of the active component, after which the suspensions are combined under the effect of ultrasound, the suspension liquid is removed and the solid particles obtained are optionally subjected to a forming step.

2. A process as claimed in the preceding claim, characterized in that the mean particle diameter of the inert components is greater than that of the active component by a factor of at least 1.1, preferably by a factor of at least 1.5 and more preferably by a factor of at least 3.5.

3. A process as claimed in any of the preceding claims, characterized in that components (A) and (I) have particle sizes of up to 1100 nm and more particularly up to 800 nm.

4. A process as claimed in any of the preceding claims, characterized in that water is used as the suspension liquid.

5. A process as claimed in any of the preceding claims, characterized in that, after separation of the solid particles, the suspension liquid is recycled to prepare suspensions of the catalytically active and/or inert components.

6. A process as claimed in any of the preceding claims, characterized in that the suspensions of solid particles of the catalytically active and/or inert components are prepared by grinding in the presence of the suspension liquid.

7. A process as claimed in the preceding claim, characterized in that the components are separately ground in a friction mill, more particularly in a ring gap mill (9).

8. A process as claimed in the preceding claim, characterized in that grinding balls are additionally used.

9. A process as claimed in claim 6 or 7, characterized in that the grinding energy introduced is at most 10 kW/l and preferably at most 3 kW/l per litre volume of material being ground.

10. A process as claimed in any of the preceding claims, characterized in that the suspensions of the fine-particle components are combined while stirring, the stirring energy introduced amounting in particular to at least 0.4 kW/l.

11. A process as claimed in any of the preceding claims, characterized in that the ultrasound acting on the suspensions of the fine-particle components while they are being combined has a power of up to 4000 W/l and preferably 200 to 600 W/l, an amplitude of 100 to 300 $\mu$m and a frequency of 16 to 30 kHz.

12. A process as claimed in any of the preceding claims, characterized in that, after removal of the suspension liquid, the solid particles obtained are dried and pelleted or extruded, optionally in the presence of a plasticizer.

13. A process as claimed in any of the preceding claims, characterized in that the suspension is subjected to spray drying and, more particularly, the granules obtained are subjected to thermal decomposition before the forming step.

14. A process as claimed in any of the preceding claims, characterized in that copper oxide (CuO) is used as the catalytically active component and titanium dioxide as the inert component.

15. A process as claimed in claim 14, characterized in that the precursor of the catalytically active component consists of copper oxide (CuO) with a mean particle diameter of 200 to 500 nm, the inert component consisting of titanium dioxide with a mean particle diameter of 600 to 1,000 nm.

16. A process as claimed in any of the preceding claims, characterized in that copper oxide (CuO), metallic copper, metallic nickel, a copper-nickel alloy and/or oxides and/or mixed oxides of nickel, cobalt, vanadium, molybdenum, iron, tin, silver, chromium, yttrium, barium, lanthanum, strontium, bismuth and/or zinc and/or metallic platinum, metallic palladium and/or metallic rhenium or a mixture or alloy/compound thereof is used as the catalytically active component.

17. A process as claimed in any of the preceding claims, characterized in that titanium dioxide, zinc oxide, silicon dioxide, zirconium dioxide, zirconium silicate and/or aluminium oxide or a mixture/compound thereof is used as the inert component.

18. A process as claimed in any of the preceding claims, characterized in that water-insoluble compounds thermally decomposable into their metal oxides are used as the catalytically active and/or inert components, hydroxides and carbonates being preferred.

## Revendications

1. Procédé de fabrication d'un catalyseur massif hétérogène gui se compose d'au moins un composant catalytiquement actif (A) et au moins d'un composant catalytiquement inerte (I), où les composants (A, I) sont dispersés l'un dans l'autre,
   caractérisé en ce qu'

   - on prbduit une ou plusieurs suspensions de particules solides du ou des composants catalytiquement actif(s) (A) et une ou plusieurs suspensions de particules solides du ou des composants catalytiquement inerte(s) (I), la granulométrie moyenne des composants inertes étant supérieure à la granulométrie moyenne du composant actif,
   - on soumet la suspension à l'action d'ultrasons,
   - on sépare le liquide de la suspension et le cas échéant
   - on soumet les particules solides obtenues à un formage.

2. Procédé selon la revendication précédente,
   caractérisé en ce que
   la granulométrie moyenne des composants inertes est au moins 1,1 fois plus grande, en particulier au moins 1,5 fois et de façon particulièrement préférée au moins 3,5 fois plus grande que la granulométrie moyenne du composant actif.

3. Procédé selon l'une des revendications précédentes,
   caractérisé en ce que
   les composants (A, I) ont des granulométries allant jusqu'à 1100 nm et en particulier jusqu'à 800 nm.

4. Procédé selon l'une des revendications précédentes,
   caractérisé en ce qu'
   on utilise l'eau comme liquide de la suspension.

5. Procédé selon l'une des revendications précédentes,
   caractérisé en ce qu'
   après la séparation des particules solides on réintroduit le liquide de la suspension pour produire des suspensions des composants catalytiquement actifs et/ou inertes.

6. Procédé selon l'une des revendications précédentes,
   caractérisé en ce qu'
   on fabrique la suspension de particules solides des composants catalytiquement actifs et/ou inertes par broyage en présence du liquide de suspension.

7. Procédé selon la revendication précédente
   caractérisé en ce qu'
   on broie les composants séparément dans un moulin à friction, en particulier dans un moulin à chambre annulaire (9).

8. Procédé selon la revendication précédente,
caractérisé en ce qu'
on utilise en outre des billes broyeuses.

9. Procédé selon la revendication 6 ou 7,
caractérisé en ce que
la puissance des billes est égale à au plus 10 kW par litre de volume de matière broyée, de préférence à au plus 3 kW/litre.

10. Procédé selon l'une des revendications précédentes,
caractérisé en ce qu'
on introduit la suspension des composants finement broyés tout en agitant, la puissance d'agitation imprimée se situant en particulier à au moins 0,4 kW/l.

11. Procédé selon l'une des revendications précédentes,
caractérisé en ce que
lorsqu'on soumet les suspensions des composants finement divisés à l'action d'ultrasons, la puissance va jusqu'à 4000 W/l, et est en particulier de 200 à 600 W/l, avec une amplitude de 100 à 300 $\mu$m et une fréquence de 16 à 30 kHz.

12. Procédé selon l'une des revendications précédentes,
caractérisé en ce qu'
après séparation du liquide de la suspension on sèche les particules solides obtenues et on les transforme en comprimés ou on les extrude, le cas échéant en ajoutant un agent plastifiant.

13. Procédé selon l'une des revendications précédentes,
caractérisé en ce qu'

- on soumet la suspension à une lyophilisation et en particulier ensuite
- on soumet encore les granulés obtenus à une décomposition thermique avant le formage.

14. Procédé selon l'une des revendications précédentes,
caractérisé en ce qu'
on utilise comme composant catalytiquement actif l'oxyde de cuivre (CuO) et comme composant inerte le dioxyde de titane.

15. Procédé selon la revendication 14,
caractérisé en ce que

- le précurseur du composant catalytiquement actif se compose d'oxyde de cuivre (CuO) ayant une granulométrie moyenne de 200 à 500 nm et
- le composant inerte se compose de dioxyde de titane ayant une granulométrie moyenne de 600 à 1000 nm.

16. Procédé selon l'une des revendications précédentes,
caractérisé en ce qu'
on utilise comme composant catalytiquement actif l'oxyde de cuivre (CuO), le cuivre métallique, le nickel métallique, un alliage cuivre-nickel et/ou les oxydes et/ou oxydes mixtes de nickel, de cobalt, de vanadium, de molybdène, de fer, d'étain, d'argent, de chrome, d'yttrium, de baryum, de lanthane, de strontium, de bismuth et/ou de zinc et/ou le platine métallique, le palladium métallique et/ou le rhénium métallique ou un de leurs mélanges ou alliages ou composés.

17. Procédé selon l'une des revendications précédentes,
caractérisé en ce qu'
on utilise comme composant inerte le dioxyde de titane, l'oxyde de zinc, le dioxyde de silicium, le dioxyde de zirconium, le silicate de zirconium et/ou l'oxyde d'aluminium ou un de leurs mélanges ou composés.

18. Procédé selon l'une des revendications précédentes,
caractérisé en ce qu'
on utilise comme composants catalytiquement actifs et/ou inertes des composés insolubles dans l'eau, décomposables thermiquement en leurs oxydes métalliques, et parmi lesquels on préfère les hydroxydes et les carbonates.

Komponente A

Korngrößenverteilung

Komponente I

Korngrößenverteilung

Mischkatalysator
(frisch)

FIG.1

FIG.2

EP 0 888 189 B1

FIG.2a

FIG.3